(19) Europäisches Patentamt
European Patent Office
Office européen des brevets

(11) **EP 2 397 850 A2**

(12) **EUROPEAN PATENT APPLICATION**
published in accordance with Art. 153(4) EPC

(43) Date of publication:
**21.12.2011 Bulletin 2011/51**

(51) Int Cl.:
*G01N 33/48* $^{(2006.01)}$     *G01N 33/53* $^{(2006.01)}$
*C12Q 1/68* $^{(2006.01)}$     *G01N 21/85* $^{(2006.01)}$
*G01N 27/26* $^{(2006.01)}$

(21) Application number: **10741369.2**

(22) Date of filing: **08.02.2010**

(86) International application number:
**PCT/KR2010/000758**

(87) International publication number:
**WO 2010/093151 (19.08.2010 Gazette 2010/33)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **10.02.2009 KR 20090010613**
**30.10.2009 KR 20090104256**

(71) Applicant: **Nanoentek, Inc.**
**Seoul 152-050 (KR)**

(72) Inventor: **HUR, Dae Sung**
**Suwon-si**
**Gyeonggi-do 440-320 (KR)**

(74) Representative: **Beck & Rössig**
**European Patent Attorneys**
**Cuvilliésstraße 14**
**81679 München (DE)**

(54) **OPEN-TYPE GROOVE CHANNEL CHIP**

(57)     The present invention relates to an open-type groove channel chip. More particularly, the present invention relates to an open-type groove channel chip comprising a main body upper plate (11), a main body lower plate (12), an injection portion (14) into which an analytical sample is to be injected, a discharge portion (13) for discharging the analytical sample, a microchannel (15) in which the analytical sample is to flow, and an open portion (16) formed at the main body of the channel chip to expose one side of the microchannel (15).

FIG. 1

EP 2 397 850 A2

**Description**

**[TECHNICAL FIELD]**

The present invention relates to an open groove channel chip, more specifically, to an open groove channel chip including a body top plate 11, a body bottom plate 12, an introduction hole 14 to introduce an analysis sample there through, an exhaustion hole 13 to exhaust the analysis sample there through, a micro-channel 15 to flow the analysis sample there in and an open part 16 formed in the body to expose a cross section area of the micro-channel 15.

**[BACKGROUND ART]**

Analysis of fluid samples has been used in various fields such as chemistry, biotechnology, pharmacy and medical science and in vitro diagnostics. Especially, there have been in progress active studies on analysis equipments with a micro size and portability, for example, lab-on-a-chip technology and point of care testing (POCT). Here, the lab-on-a-chip technology realizes on a microchip various experiment processes including separation, purification, mixture, labeling, analysis and cleaning processes, which are performed in a laboratory, by using microfluidics. The point of care testing (POCT) is a diagnostic tool which allows a complex testing of blood, body fluid performed in a hospital or laboratory to be performed on the spot or by an individual easily.

In addition, usage and studies on flow cytometry used to figure out dispersion of cells having a specific protein have been in progress. The flow cytometry is technology which figures out physiology and biochemical analysis of each cell in a shorter time based on cell sizes, cell distribution, and cell functions recognized by fluorescent dyes by using all of hydraulic, optical and electronic technologies.

Such the flow cytometry determines "an immune state" possessed by a single cell via a fluorescent dyes and a monoclonal antibody and it enables studies on various immune diseases. Also, the flow cytometry helps to be set a process of diagnosis or treatment is set after determining a characteristic of a malignant tumor based on analysis of DNA contents in a cell or nucleus and it helps a prognosis of the malignant tumor to be observed. In addition, the flow cytometry is used in chromosome study, reticulocyte determination, calcium measurement in a cell and microorganism test. Application range of the flow cytometry has been getting broadened.

Those various diagnosis equipments which use the fluid samples utilizes relating technologies such as microfluidics and microfluidic manuplating, and they are operated by a micromotor or capillary used as driving force after moving the fluid samples via a micro-channel formed in a chip.

However, it is not easy to process corner areas of the micro-channel uniformly without damage, when manufacturing a minute closed channel having a size of dozens of micrometers. This might result in an error generated in standards used in mass-production and quality management. Also, the minute error of the channel structure might interfere with fluid flow and might result in the result of the analysis without consistency.

Especially, according to a groove channel chip including multichannel proper to detection of a particle with a low concentration or counting of rare cells or bacteria in a sample, it is difficult to adhere a top plate to a bottom plate of the multichannel completely because of limits of a manufacturing technology. As a result, regular gaps cannot be occur between the multichannel and analysis sample might leak aside or a capillary source might deteriorate, only to generate a problem of failing to flow the sample fluid properly.

In addition, groove channel chips having conventional micro-channels have the closed structure. Because of that, selection of materials used for maintaining transparency of a body is limited and detection of an analysis sample which passing through the channel is limited.

In the meanwhile, it is not easy to perform an experiment to measure a small amount of fluid flowing along the micro-channel because of the micro-small scale. Even if the amount of the fluid is measured, the sensibility of a measuring device has to be high and equipments with a high price are required.

Also, it is important to reduce loss of lights incident on a measuring surface to enhance efficiency of optical measurement in a micro-sized analysis system. An observing object such as blood and a cell is measured by an optical device, while moving along the micro-channel. At this time, the loss of lights might be generated.

As a result, the inventor of the present invention invents an open groove channel which can minimize influence of a micro-channel structure on fluid flow and eliminate leakage of the analysis sample generated between neighboring channels or capillary deterioration, especially, which can minimize limit on selection of a material or detection of an analysis sample as well as.

**[DISCLOSURE OF INVENTION]**

**[TECHNICAL PROBLEM]**

To solve the problems, an object of the present invention is to provide a grove channel chip which minimize influence of a micro-channel structure on fluid flow and eliminate leakage of the analysis sample generated between neighboring channels or capillary deterioration,

Another object of the present invention is to provide a groove channel chip which can minimize limit on selection of a material or detection of an analysis sample as well as.

**[TECHNICAL SOLUTION]**

To achieve these objects and other advantages and in accordance with the purpose of the invention, as embodied and broadly described herein, an open type groove channel chip includes a top plate 11 of a body; a bottom plate 12 of the body; an introduction part 14 in which an analysis sample is introduced; an exhaustion part 13 through which the analysis sample is exhausted; a micro-channel 15 where the analysis sample is flowing; and an open part 16 formed in the body to expose a cross-section of the micro-channel 15.

The open part 16 is located in the top plate 11 or the bottom plate 12 of the body.

An optical sensor may be located in the open part 16 to sense an optical signal of the analysis sample passing the micro-channel.

The micro-channel 15 may be configured of multi-channels.

A surface of the micro-channel 15 may be surface-treated to allow the micro-channel 15 to adjust a flow rate by plasma surface modification or by chemical reforming of the surface.

The depth of the micro-channel 15 may be $1 \sim 500\ \mu m$ or the width of the micro-channel 15 may be $1 \sim 500\ \mu m$.

Labeling reaction of the analysis sample or antigen-antibody specific reaction of the analysis sample may be performed in the introduction part 14.

The exhaustion part 13 may include an expansion part 21 in which the analysis sample having passed the micro-channel 15 is collected or a multi-channel part 22 along which the analysis sample having passed the micro-channel 15 is flowing in a radial direction.

The exhaustion part 13 further comprises a detection part 31 to detect the analysis sample having passed the micro-channel or a sensor 32 to detect the analysis sample.

**[ADVANTAGEOUS EFFECTS]**

The present invention has following advantageous effects.

According to the open type groove channel chip, the capillary flow is generated in the open type micro-channel having the surface treatment, without an external driving force, and the sample solution may flow along the channel. As a result, an external driving force source such as a pump is not needed and loss of lights generated by a substrate is reduced. Also, the open type groove channel chip according to the present invention has advantages of the reduced light loss and of detecting samples in various channels simultaneously.

Furthermore, the influence of the micro-channel structure on the fluid flow may be minimized. Especially, leakage of the analysis sample to neighboring channels in the multi-micro-channel or deterioration of the capillary force may be prevented. As a result, the open type groove channel chip according to the present invention is proper when detecting low concentrated particles or counting rare cells or bacteria in the sample.

Still further, the entire manufacturing process of the groove channel chip may be efficient and simple. The cross section of the micro-channel is exposed outside. As a result, limit on material selection of the channel chip or detection of the analysis sample may be reduced as much as possible.

Still further, the adjustment of the contact angle and variation of the channel shape by the surface treatment performed in manufacturing the channel may allow the open type groove channel chip according to the present invention may be utilized in manufacturing a micro-analysis system which uses various samples.

**[BRIEF DESCRIPTION OF THE DRAWINGS]**

The accompanying drawings, which are included to provide further understanding of the disclosure and are incorporated in and constitute a part of this application, illustrate embodiments of the disclosure and together with the description serve to explain the principle of the disclosure.

In the drawings:

FIGS. 1 and 2 are a perspective view and a front projection view illustrating a groove channel chip according to the present invention;

FIG. 3 is a perspective view illustrating a top plate of the groove channel chip;

FIG. 4 is a photograph of flow of a particle fluorescence-labeled in fluid observed at an open part of the groove channel chip according to the present invention;

FIG. 5 is an enlarged view illustrating a multi-micro-channel of the groove channel chip according to the present invention;

FIG. 6 is a photograph of particles moving along channel flow after sample solution of 20 $\mu\ell$ having a fluorescent particle with a diameter of 6 $\mu$m therein is put in the channel chip manufactured as shown in FIG. 1;

FIG. 7 is a graph illustrating the velocity of the particle measured based on the time by using a particle detecting method in the channel chip manufactured as shown in FIG. 1;

FIG. 8 is a diagram illustrating comparison of meniscuses formed in the channels by performing computational fluid dynamics (CFD) for a single channel with a top plate and a single channel without a top plate;

FIG. 9 is a diagram illustrating a process of the meniscus formed in the channel by performing the computational fluid dynamics (CFD) for the single channel without the top plate;

FIGS. 10 and 11 are diagrams illustrating fluid contact angles of a plastic chip before and after plasma surface treatment;

FIG. 12 is a diagram illustrating an embodiment of an appearance of an exhaustion part provided in the groove channel chip according to the present invention; and

FIG. 13 is a diagram illustrating another embodiment of the appearance of the exhaustion part provided in the groove channel chip according to the present invention.


**[BEST MODE]**

As follows, a groove channel chip according to the present invention will be described in detail in reference to the accompanying drawings.

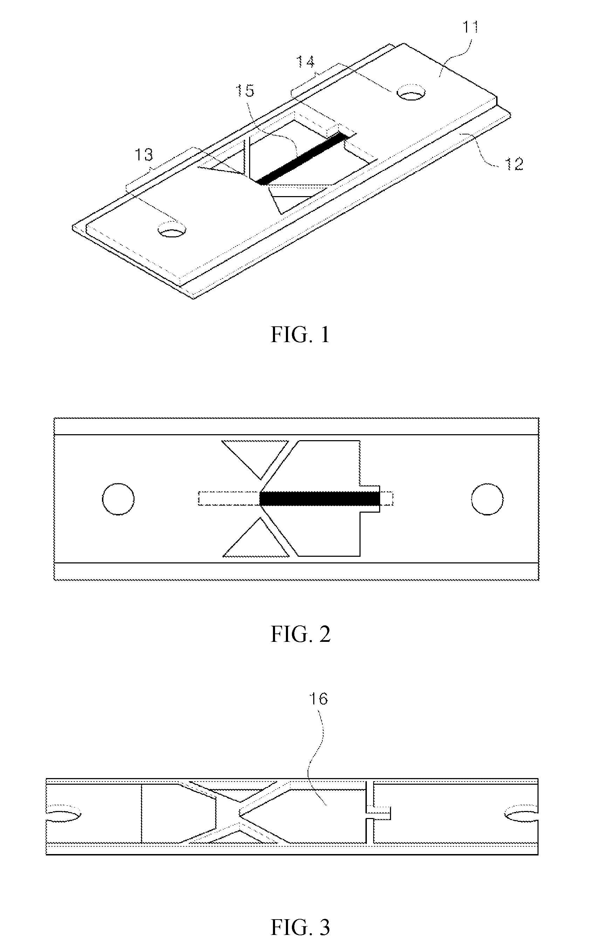

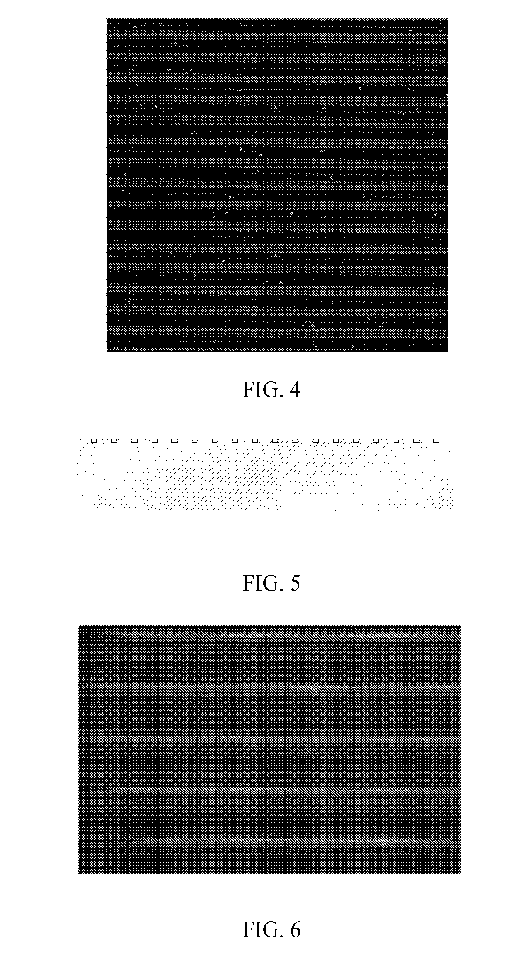

FIGS. 1 and 2 are a perspective view and a front projection view illustrating a groove channel chip according to the present invention. FIG. 3 is a perspective view illustrating a top plate of the groove channel chip. FIG. 4 is a photograph of flow of a particle fluorescence-labeled in fluid observed at an open part of the groove channel chip according to the present invention.

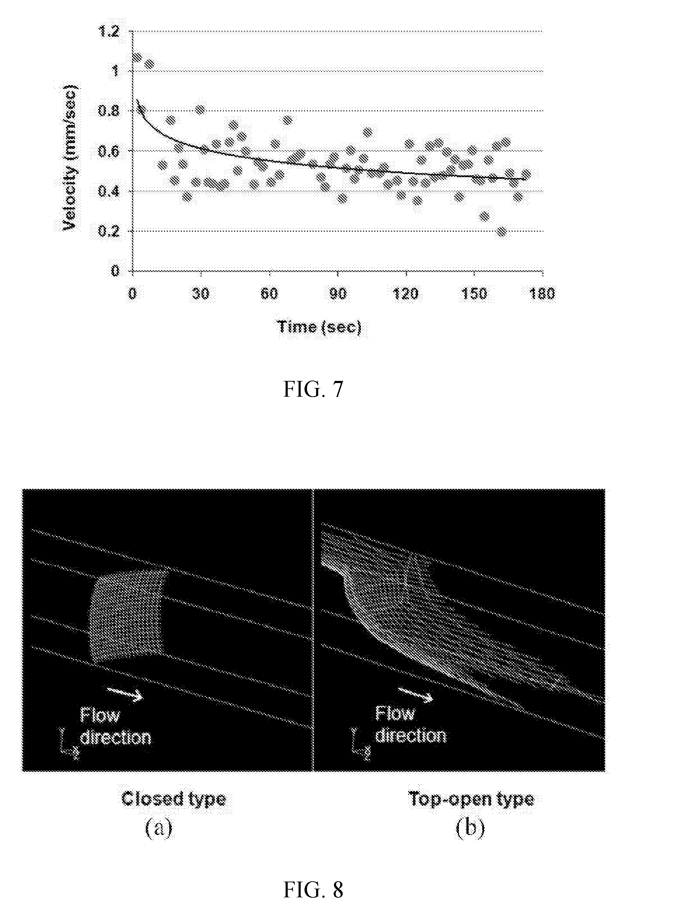

FIG. 5 is an enlarged view illustrating a multi-micro-channel of the groove channel chip according to the present invention. FIG. 6 is a photograph of particles moving along channel flow after sample solution of 20 $\mu\ell$ having a fluorescent particle with a diameter of 6 $\mu$m therein is put in the channel chip manufactured as shown in FIG. 1. FIG. 7 is a graph illustrating the velocity of the particle measured based on the time by using a particle detecting method in the channel chip manufactured as shown in FIG. 1.

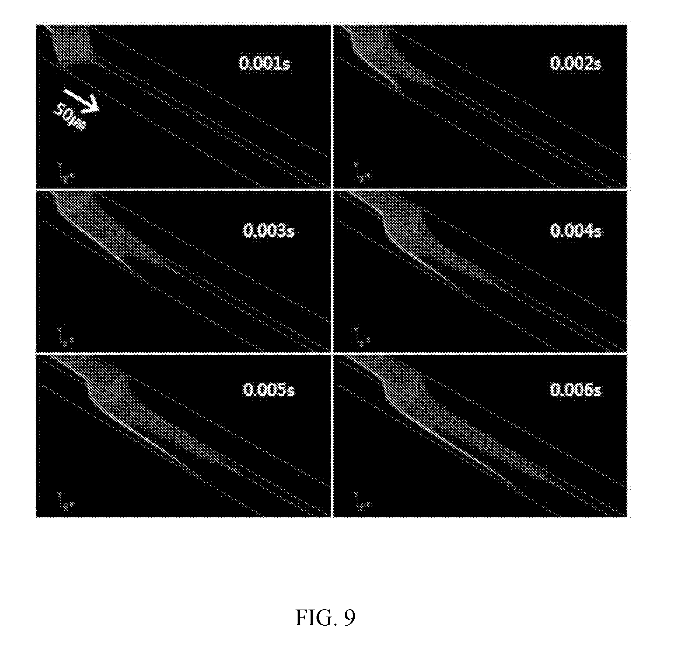

FIG. 8 is a diagram illustrating comparison of meniscuses formed in the channels by performing computational fluid dynamics (CFD) for a single channel with a top plate and a single channel without a top plate. FIG. 9 is a diagram illustrating a process of the meniscus formed in the channel by performing the computational fluid dynamics (CFD) for the single channel without the top plate.

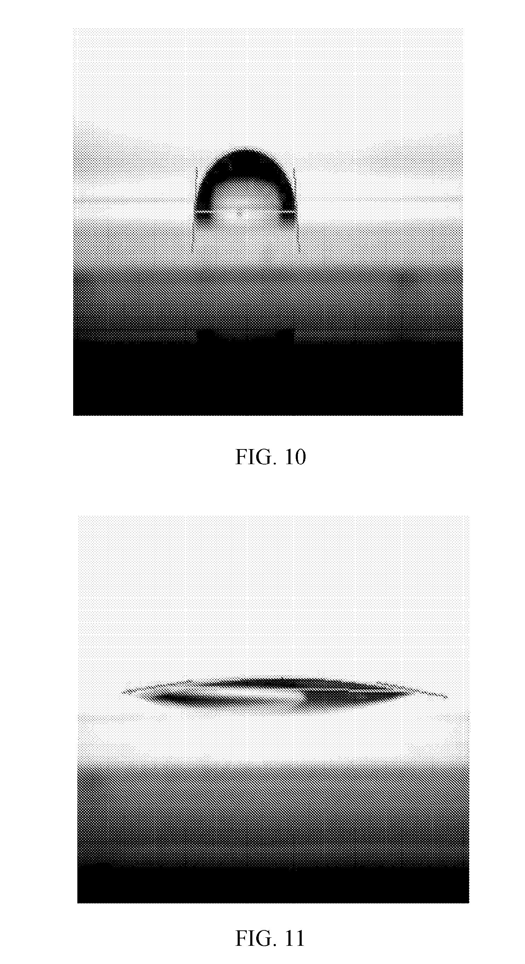

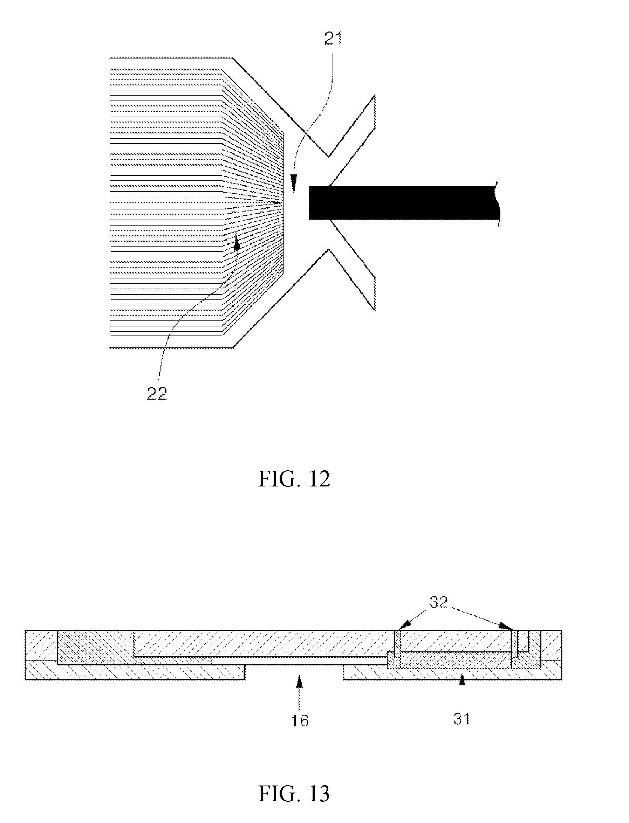

FIGS. 10 and 11 are diagrams illustrating fluid contact angles of a plastic chip before and after plasma surface treatment. FIG. 12 is a diagram illustrating an embodiment of an appearance of an exhaustion part provided in the groove channel chip according to the present invention. FIG. 13 is a diagram illustrating another embodiment of the appearance of the exhaustion part provided in the groove channel chip according to the present invention.

A flow cell according to the present invention includes a top body plate 11, a bottom body plate 12, an introduction part 14 to introduce an analysis sample there through, an exhaustion part 14 to exhaust the analysis sample there through, a micro-channel 15 where the analysis sample is flowing, and an open part 16 formed in a body to expose a cross-section area of the micro-channel 15 outside.

Once the analysis sample is introduced into the cell via the introduction part, a desired particle with low concentration provided in the analysis sample is detected while passing the introduction part 14 or the analysis sample may react with a reaction reagent to count rare cells or bacteria provided in the analysis sample.

Generally, to detect the desired particle, specific reaction of the analysis sample may be performed in the introduction part 14 based on labeling reaction of the analysis sample using a fluorescent material or antigen-antibody reaction, to detect the desired particle.

In other words, various detection means such as a sensor may selectively identify only the desired material based on hybridization of a DNA or RNA aptamer or specification of antigen-antibody possessed by a protein.

The labeled analysis sample passes the micro-channel 15. At this time, the open part 16 is formed in the body to expose a cross section area of the micro-channel. FIG. 4 is a photograph of flow of the fluorescently labeled particle in fluid observed at the open part. The open part 16 allows visible identified labels such as fluorescent label to be identified directly or an optical sensor is installed in the open part to detect labels. If necessary, beads or detection particles may be arranged in the micro-channel 15 regularly for the detection.

Based on the result of the detection, it is identified that the open micro-channel has increased optical detection efficiency up to approximately 10% ~ 15% because of its structural characteristic, in comparison to a closed type micro-channel. The open part 16 may be located in the top plate of the body or the bottom plate 12 of the body.

As the open micro-channel is used, the fluid may be prevented from leaking via a gap between the top and bottom plates because of incomplete adhesion between the top and bottom plates of the micro-channel.

When a plurality of multi-channels formed in the channel part as shown in FIG. 5 are used, the phenomenon of fluid leakage toward neighboring channels or the phenomenon of capillary force deterioration may be reduced effectively, compared with the multi-channels formed by using the closed micro-channel. At this time, it is preferable that the depth of the micro-channels may be in a range of 1 ~ 500 $\mu$m and that the width of the micro-channels is 1 ~ 500 $\mu$m.

In the meanwhile, a plastic material of the micro-channel may be transparent or opaque because of the open type structure. Specifically, all types of useable conventional plastic materials may be used such as polystyrene (PS), polycarbonate (PC) or polymethylmethacrylate (PMMA). A surface of the plastic may be modified in a plasma treatment process or a chemical treatment process to adjust a flow rate and maintain a capillary force.

It is preferable that the surface of the plastic material is modified in the plasma treatment process to maintain the surface modification effect constantly. At this time, it is more preferable that the polystyrene (PS) is used as plastic material to enhance the constant surface modification effect. A contact angle of the surface of the plastic chip is treated to be approximately 10° or less, to prepare the open type multichannel.

Following embodiments 1 through 4 will describe a method of manufacturing an open type channel chip and fluid flow by using the channel, which will be analyzed based on flow analysis of computational fluid dynamics. Also, a method of manufacturing an open type micro-channel having a surface of which a modification treatment is performed will be described.

[Embodiment 1] Method of Manufacturing Open Type Groove Channel and Measuring Channel Flow:

A photosensitizer is coated on a silicon wafer to be high as a channel. After that, ultraviolet rays are projected to a photomask placed on the photosensitizer to make a desired shape. A metal material capable of flow electric currents therein is deposited thin and a stamper which will be used in molding is fabricated in a nickel plating bath. The thickness of the plated stamper is uniform in a planarization process.

Hence, the plated stamper is diced to be proper to a mold of the molding equipment in a dicing process and the diced stamper is mounted in the mold of the molding equipment. Open type rectangular cross section plastic micro-channel is manufactured, with an injection part capable of receiving sample solution of 20 $\mu\ell$ via a injection mold and channels placed in parallel with a width of 20 $\mu$m, a height of 20mm and a height of 20 $\mu$m.

The sample solution of 20 $\mu\ell$ having fluorescent particles with a diameter of 6 $\mu$m is poured in the injection part and the particles flowing along channel flow are photographed as shown in FIG. 6. Images taken in a resolution of 1280 $\times$ 024 pixels, a frame rate of 5.44Hz and an exposure time of 60ms by a fluorescent microscope (IX71, Olympus) and a low light level ICCD camera (Dicam-Pro, PCO) are analyzed to measure a flow rate.

FIG. 7 illustrates the velocity of the particles which is measured based on the time by using a particle detection method after the sample is injected into a channel inlet part. In an initial period, the velocity is drastically decreasing and gently decreasing after that. The flow of the particles is stabilized and the rate distribution is 0.4 - 0.6mm/s.

Most amount of the solution is collected in a channel bottom area in a predetermined time after the injected sample solution of 20 $\mu\ell$ moving toward an outlet part along the channel. The movement of the sample solution changes the direction of the capillary force. In this case, the solution collected in the outlet part is moved toward the inlet part.

[Embodiment 2] Computational Fluid Dynamics Flow Analysis:

A commercially used S/W fluent is used to perform computational fluid dynamics analysis. A top plate open type rectangular cross section single micro-channel lattice is made and water is injected in an inlet part. After that, it is analyzed as abnormal flow to check a time based state and a volume of fluid (VOF) model is used not to mix gas and liquid with each other.

When the water is injected in the inlet part, the pressure at this time is calculated by using a pressure difference between interfaces of a rectangular channel based on Young's relational expression proposed by Ichikawa (Ichikawa, N, etc., 2004, "Interface Motion of Capillary-driven Flow in Rectangular Micro-channel", J, Colloid Interface Sci., Vol. 280, No.1, pp.155-164) as shown in a following formula (1):

$$\triangle P = \sigma\left(\frac{1}{R_w} + \frac{1}{R_h}\right)$$

$$R_w = \frac{w}{2\cos\theta}, \quad R_h = \frac{h}{2\cos\theta}.$$

'w' refers to the width of the channel and 'h' refers to the height of the channel. 'σ' refers to a surface tension of the water and 'θ' refers to a contact angle. The pressure calculated based on the above formula (1) is an initial condition when the water is injected. As the top plate is not provided, an upper surface and an outlet part of the channel are in a standby state and the calculation is performed at a time interval of $10^{-5}$s.

Based on a channel having the same size and shape as the channel with the width and the height of 20 μm and the length of 50mm used in an actual experiment, flow in the same sized channel with the top plate and flow in the 10 μm width and 4 μm height channel without the top plate are compared and analyzed.

Computational fluid dynamics analysis for the single channel having the same sized cross section as the channel having the width of 20 μm and the length of 50mm used in the particle detection rate measuring experiment and the single channel without the top plate. Meniscus formed in each channel is compared as shown in FIG. 8.

Fluid is moving in symmetry along X axis and Y axis with respect to a center of a perpendicularly cross section in the channel having the width and height of 20 μm with the top plate. The liquid is moving at two lower corners firstly in the channel without the top plate. The liquid moves most rapidly at corners in the rectangular channel, regardless of the existence of the top plate.

There is not so much difference of the liquid velocity between a center and corners in the channel with the top plate. In contrast, wetting of the upper surface is not generated in the channel without the top plate and there is increasing difference between the velocity at the corners and at the center as shown in FIG. 9. In other words, an end of the meniscus of the water moving along the corners flows along the channel at the highest velocity and the velocity is different from the velocity of the water moving while filling the channel.

The velocity of the fluorescent particle measured in the particle detection velocity measurement experiment has to be compared with the velocity of the fluid filling the channel, not the moving velocity of the end of the meniscus, which is shown in Table 1. The existence of the channel top plate affects the entire flow rate. When the top plate is provided, the time taken for the meniscus of the water to reach the end of the channel is shorter ten times as shorter as the time when the top plate is not provided.

To identify the influence of the channel cross section size on the movement of the fluid, table 1 shows comparison of movement of fluid in channels having a width and a height which are differentiated to be 10 μm, 20 μm and 40 μm.

The fluid flows in a 'V' shape toward the outlet part at a bottom surface of the channel without the top plate. The position where the liquid fills the channel is determined as an angular point of the 'V' shape and the position of the liquid for each channel is compared with an average velocity.

[Table 1]

| Channel Width & Height | Total Distance of Movement For 5 sec. | Average Velocity of Water (mm/S) |
| --- | --- | --- |
| 10 | 0.81 | 0.16 |
| 20 | 2.00 | 0.4 |
| 40 | 2.35 | 0.47 |

As shown in FIG. 1, the location of the meniscus of the water filling the channel in a predetermined time based on computational fluid dynamics is moving more rapidly as the cross sectional size of the channel is increasing in a range of 10 ∼ 40 μm. When the size of the channel is 20 μm and 40 μm, the difference between the movement velocities is not so large. When the size of the channel is 10 μm, it is shown that the movement velocity is decreased by 50% or more.

The entire meniscus shapes are similar in all of the channels and the propulsion force of the water is different in each of the channels. In general, according to a macroscopic capillary phenomenon which is able to be seen, the height of the water moving upwardly or downwardly along a capillary pipe is increasing as the capillary pipe is smaller.

In other words, the height of a liquid column is in reverse proportion to a radius.

However, in case the size of the channel is very small and a small amount of liquid is moving in the channel, in other words, in case the influence of the gravity is little, the phenomenon varies. In case of a circular pipe having a diameter of hundreds of $\mu$m or less, Washburn and Kim proposes that a moving distance is getting longer as a diameter of the capillary pipe is getting large (Washburn, E, W., 1921, "The Dynamics of Capillary Flow, "Phys, Rev., Vol. 17, No. 3, pp. 273-283., Kim, E. and Whitesides, G. M., 1997, "Imbibition and Flow of Wetting Liquids in Noncircular Capillaries," J Phys. Chem..B, Vol. 101, No. 6, pp. 855-863).

A force dominating the movement of the fluid varies between a macroscopic unit boundary and a microscopic unit boundary. The present study identifies by using computational fluid dynamics that the propulsion force of the water is getting large in a range of dozens of $\mu$m as the size of the channel is getting large, in case of the rectangular cross section channel without the top plate.

[Embodiment 3] Method of Groove Channel Chip in Vacuum Plasma Treatment Process:

1) Plastic Chip Cleaning Process:

After a predetermined amount of detergent is added to an ultrasonic cleaner, a plastic chip having an open channel is cleaned in the ultrasonic cleaner for 3 minutes. After that, the cleaned plastic chip is showered in deionized (D.I) water. The plastic chip is put into the ultrasonic cleaner and it is cleaned for 10 minutes. After that, the cleaned plastic chip is re-showered in the deionized (D.I) water. The plastic chip is dried in a micro-oven to remove the moisture contained in the plastic chip.

2) Vacuum Oxygen Plasma Treatment Process:

A RF power, the amount of oxygen inflow into a plasma treatment equipment chamber and a plasma treatment time are prepared to have following conditions:

[Table 2]

| RF Power | 350W |
| --- | --- |
| O2 Inflow | 150 sccm |
| Treatment Time | 300 c |

The chip cleaned in the step 1) is put into a chamber provided in a plasma system. The plasma system set to have the above conditions is operated to perform plasma treatment. After the plasma treatment, the chip is taken out and it is put in a slide box.

3) Result of Contact Angle Measurement after Surface Treatment:

As shown in FIG. 10, a fluid contact angle of the plastic chip before the surface treatment is 87.9°. As shown in FIG. 11, a fluid contact angle of the plastic chip after the surface treatment is 7.6°. The surface contact angle of the plastic chip is treated to be approximately 10° or less, and multi open micro-channels may be provided.

[Embodiment 4] Manufacturing of Groove channel Chip using Chemical Surface Treatment:

A surface active agent is coated on a surface of the plastic chip having the chemical surface treatment and the plastic chip dried completely is prepared. Like the embodiment 3, a fluid contact angle of the coated plastic chip surface is treated to be 10°or less, to prepare a multi open micro-channels.

The analysis sample having passed the micro-channel manufactured according to the embodiments is moved to the exhaustion part 13. At this time, the exhaustion part 13 may include an expansion part 21 configured to collect the analysis sample having passed the micro-channel 15 and it may include a multi-channel part 22 to re-flow the fluid collected in the expanded part in a radial direction, as shown in FIG. 12.

At this time, the capillary force is increased entirely while the fluid passing a three-surface structure of the open micro-channel is drawn into the closed expansion part. This may be an impetus for flowing the fluid along the groove channel chip at a constant velocity for a preset time period.

In addition, as shown in FIG. 13, a detection part 31 may be further provided in the exhaustion part to detect the analysis sample having passed the micro-channel. At this time, the detection part may include a sensor 32 to detect the analysis sample. At this time, the sensor may be an optical sensor configured to sense an optical signal within the detection part or an electrochemical sensor configured to sense an electrochemical signal within the detection part.

As described above, the capillary pipe flow without external driving force is generated in the open micro-channel having the surface treatment and the sample solution may flow along the channel. When such the open micro-channel is used in an optical detection type micro-analyzer, an external power supply source such as a pump does not have to be provided. In addition, loss of lights generated by a substrate may be reduced advantageously and it may be possible to detect the samples in the channels simultaneously, which is another advantage.

Also, when manufacturing the channel, the surface treatment is performed such that the contact angle may be adjusted and that the shape of the channels may be varied. As a result, the present invention may be utilized in manufacturing a micro-analysis system using various samples.

It will be apparent to those skilled in the art that various modifications and variations can be made in the present invention without departing from the spirit or scope of the invention. Thus, it is intended that the present invention cover the modifications and variations of this invention provided they come within the scope of the appended claims and their equivalents.

**Claims**

1.  An open type groove channel chip comprising:

    a top plate 11 of a body;
    a bottom plate 12 of the body;
    an introduction part 14 in which an analysis sample is introduced;
    an exhaustion part 13 through which the analysis sample is exhausted;
    a micro-channel 15 where the analysis sample is flowing; and
    an open part 16 formed in the body to expose a cross-section of the micro-channel 15.

2.  The open type groove channel chip as claimed in claim 1, wherein the open part 16 is located in the top plate 11 of the body.

3.  The open type groove channel chip as claimed in claim 1, wherein the open part 16 is located in the bottom plate 12 of the body.

4.  The open type groove channel chip as claimed in claim 1, wherein an optical sensor is located in the open part 16 to sense an optical signal of the analysis sample passing the micro-channel.

5.  The open type groove channel chip as claimed in claim 1, wherein the micro-channel 15 is configured of multi-channels.

6.  The open type groove channel chip as claimed in claim 1, wherein a surface of the micro-channel 15 is surface-treated to allow the micro-channel 15 to adjust a flow rate.

7.  The open type groove channel chip as claimed in claim 6, wherein the surface treatment is performed by plasma surface modification.

8.  The open type groove channel chip as claimed in claim 7, wherein the groove channel chip is formed of polystyrene (PS).

9.  The open type groove channel chip as claimed in claim 6, wherein the surface treatment is performed by chemical reforming of the surface.

10. The open type groove channel chip as claimed in claim 1, wherein the depth of the micro-channel 15 is 1 ~ 500 μm.

11. The open type groove channel chip as claimed in claim 1, wherein the width of the micro-channel 15 is 1 ~ 500 μm.

12. The open type groove channel chip as claimed in claim 1, wherein beads are arranged in the micro-channel 15.

13. The open type groove channel chip as claimed in claim 1, wherein labeling reaction of the analysis sample is performed in the introduction part 14.

14. The open type groove channel chip as claimed in claim 1, wherein antigen-antibody specific reaction of the analysis sample is performed in the introduction part 14.

15. The open type groove channel chip as claimed in claim 1, wherein hybridization of DNA or RNA aptamers is performed in the introduction part 14.

16. The open type groove channel chip as claimed in claim 1, wherein the exhaustion part 13 comprises an expansion part 21 in which the analysis sample having passed the micro-channel 15 is collected.

17. The open type groove channel chip as claimed in claim 1, wherein the exhaustion part 13 comprises a multi-channel part 22 along which the analysis sample having passed the micro-channel 15 is flowing in a radial direction.

18. The open type groove channel chip as claimed in claim 1, wherein the exhaustion part 13 further comprises a detection part 31 to detect the analysis sample having passed the micro-channel.

19. The open type groove channel chip as claimed in claim 18, wherein the detection part 31 further comprises a sensor 32 to detect the analysis sample.

20. The open type groove channel chip as claimed in claim 19, wherein the sensor 32 is an optical sensor configured to sense an optical signal within the detection part 31.

21. The open type groove channel chip as claimed in claim 19, wherein the sensor 32 is an electrochemical sensor configured to sense an electrochemical signal within the detection part 31.

FIG. 1

FIG. 2

FIG. 3

FIG. 4

FIG. 5

FIG. 6

FIG. 7

Closed type
(a)

Top-open type
(b)

FIG. 8

FIG. 9

FIG. 10

FIG. 11

FIG. 12

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Ichikawa, N.** Interface Motion of Capillary-driven Flow in Rectangular Micro-channel. *J, Colloid Interface Sci.,* 2004, vol. 280 (1), 155-164
- **Washburn, E, W.** The Dynamics of Capillary Flow. *Phys, Rev.,* 1921, vol. 17 (3), 273-283

- **Kim, E. ; Whitesides, G. M.** Imbibition and Flow of Wetting Liquids in Noncircular Capillaries. *J Phys. Chem..B,* 1997, vol. 101 (6), 855-863